# EUROPÄISCHE PATENTSCHRIFT

(11) **EP 1 126 815 B1**
(45) Veröffentlichungstag und Bekanntmachung des Hinweises auf die Patenterteilung: **04.02.2004**
(21) Anmeldenummer: 99960888.8
(22) Anmeldetag: 04.11.1999
(51) Int. Cl.: A61K 7/027, A61K 7/00

(54) **VITAMINHALTIGER LIPPEN- ODER PFLEGESTIFT**
LIP OR CARE STICK WHICH CONTAINS VITAMINS
BATON DE ROUGE A LEVRES OU BATON POUR SOINS DES LEVRES, RENFERMANT DES VITAMINES

(30) Priorität: 04.11.1998 DE 19852196
(43) Veröffentlichungstag der Anmeldung: 29.08.2001
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC); JOLY, Benoit, F-06700 Saint Laurent du Var (FR)
(74) Vertreter: Walter, Wolf-Jürgen
(86) Internationale Anmeldenummer: PCT/DE1999/003585
(87) Internationale Veröffentlichungsnummer: WO 2000/025733

(56) Entgegenhaltungen:
- EP-A- 0 974 332
- WO-A-97/13497
- DE-U- 29 717 497

## Beschreibung

Die Erfindung betrifft einen vitaminhaltigen Lippen- oder Pflegestift für die kosmetische Anwendung.

Aus der DE 38 206 93 A1 ist bekannt, daß bei topischen Zubereitungen freie Tocopherole in Anwesenheit von UV-Lichtschutzmitteln durch Zusätze von Ascorbinsäuren von C₁₂-C₁₈-Fettsäuren und Citronensäureestern von Partialglyceriden von C₁₂-C₂₀-Fettsäuren stabilisiert werden können.

Aus der US-A-4954332 ist ebenfalls bekannt, daß sich Tocopherole und Vitamin C bei Vorhandensein von UV-Filtern zersetzen, und es werden zur Vermeidung der Zersetzung steroide und nichtsteroide entzündungswidrige Mittel hinzugegeben.

Es sind weiterhin eine Reihe von Lippenstiftprodukten bekannt, die als Antioxidationsmittel Zusätze von Tocopherol enthalten ( z.B. US-A-4699780).

Der Erfindung liegt die Aufgabe zugrunde, neue Lippen- und Pflegestifte zu entwickeln mit einem Gehalt an stabilisierten Tocopherolen.

Eine weitere Aufgabe der Erfindung besteht darin, den Gehalt an Tocopherolen neben üblichen chemischen Lichtschutzmitteln auf einem stabilen Niveau zu halten.

Erfindungsgemäß bereitgestellt werden Lippen- oder Pflegestifte auf Wachsbasis, bei denen die kosmetische stiftmasse einen Anlagerungskomplex enthält, bestehend aus einem Cellulosederivat, umfassend Carboxymethylcellulose, Methylcellulose, Hydroxymethylcellulose, Hydroxypropylcellulose und Hydroxypropylmethylcellulose, mit einer Teilchengröße von 0,5 bis 100 µm, einem Phospholipid und einem α-Tocopherolester, umfassend Tocopherylacetat, -succinat, -propionat, -oleat, -linolat, -sorbat, wobei der Anteil des Komplexes 0,5 bis 40 Gew-% beträgt, bezogen auf die Gesamtmasse, zusammen mit kosmetisch üblichen Fetten, Wachsen und Zusatzstoffen, die einen Anteil von 99,5 bis 60 Gew-% haben.

Es wurde gefunden, daß die Anlagerungskomplexe von Tocopherol, einem Phospholipid und Cellulosederivaten eine Form darstellen, bei der die Cellulosekomplexe durch Feuchtigkeit gelöst und zusammen mit den angelagerten Tocopherolen vom Körper des Anwenders aufgenommen werden, und das Tocopherol als Vitamin im Körper verarbeitet werden kann.

Ein bevorzugter α-Tocopherolester ist α-Tocopherylacetat.

Der Anteil des Anlagerungskomplexes beträgt vorzugsweise 2 bis 30 Gew-%, vorzugsweise 5 bis 25 Gew-%, bezogen auf die Gesamtmasse. Weitere bevorzugte Gehalte liegen bei 8-28 Gew-%, insbesondere 8-18 Gew-%.

Als Phospholipid eignen sich Phosphatidylcholin, Phosphatidylethanolamin, Phosphatidylinositol sowie Phosphatidylserin und Gemische davon. Besonders bevorzugt ist Phosphatidylcholin. Der Gehalt an Phospholipiden kann im Bereich von 0,01 bis 5 Gew-%, vorzugsweise 0,05 bis 3 Gew-% liegen, bezogen auf die Gesamtzusammensetzung.

Weitere Zusatzstoffe sind ausgewählt unter öllöslichen UVB-Filtern. Dazu gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher. Bevorzugte UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Es wurde gefunden, daß der Gehalt an diesen UV-Filtern keine nachteiligen Wirkungen auf den Tocopherolgehalt ausübt, d.h. der Tocopherolgehalt bleibt im wesentlichen stabil, und es treten keine aus dem Stand der Technik bekannte Zersetzungserscheinungen auf.

Die organischen UV-Filter können im Bereich von 2 bis 15 Gew-% in der Gesamtmasse des Stiftes enthalten sein.

Als weitere Zusätzstoffe können auch anorganische UV-Filter ausgewählt werden, wie TiO₂, SiO₂, ZnO und Gemischen davon, wobei deren Gehalte der gewünschten Färbung des Stiftes angepaßt werden müssen. Anteile von 1-4 Gew-% TiO₂ sind vorteilhaft.

Die Wachse können ausgewählt werden unter Carnaubawachs, Candellilawachs, ozokerit, Bienenwachs, Montanwachs, Wollwachs, Ceresin, Mikrowachsen, Paraffinwachsen, Petrolatum.

Weitere Zusatzstoffe sind beispielsweise ausgewählt unter Rizinusöl, Paraffinöl, Myristyllaktat, Isopropylmyristat, Isopropyllanolat, Isopropylpalmitat, p-Hydroxybenzoesäurepropylester.

Geeignete öle sind beispielsweise auch Mineralöle, Hydrogenated Polyisobuten, Polyisopren, Squalane, Tridecyltrimellitat, Trimethylpropan-triisostearat, Isodecylcitrat, Neopentylglycoldiheptanoat, PPG-15-stearylether, Calendulaöl, Jojobaöl, Avocadoöl, Macadamianußöl, Olivenöl oder ein Gemisch mehrerer davon. Je nachdem welche öle ausgewählt werden, werden die kosmetischen Eigenschaften der festen Zusammensetzung beeinflußt, wie Weichheit, Härte, Spreitungswirkung.

Zur Verbesserung der Festigkeit und Stabilität der erfindungsgemäßen Stifte dient der Gehalt von Polymeren oder Copolymeren, wie beispielsweise von hydrierten Styren/Methylstyren/Inden-Copolymeren, z.B. Régalite R101® von Hercules; Copolymeren von Vinylpyrrolidon und langkettigen α-Olefinen, wie Antaron V220®, Antaron V216®, Unimer U15®; Salzen von Fettsäureestern, wie Sodium Isostearoyl lactylate (Pationic ISL® von Rita Corp.); und PEG-120 Methyl glucose dioleate (z.B. Glucamate DOE 120®); sowie Gemischen davon, sowie von Kautschuken, wie Polyisopren.

Bevorzugt sind auch hydrierte Styren/Methylstyren/Inden-Copolymere, da sie die Bruchfestigkeit von Stiften mindern. Außerdem gewährleisten hydrierte Kohlenwasserstoffharze eine bessere Formbarkeit und Einfüllbarkeit der Zusammensetzung in Lippenstift- oder dünne Stiftformen.

Weitere Zusatz- bzw. Wirkstoffe in den kosmetischen Znsammensetzungen können sein Vitamine, z.B. Vitamin A oder Vitamin A-Derivate; gefärbte Pflanzenextrakte, wie fettlöslicher Gardenienextrakt, fettlöslicher Karottenextrakt, Paprika-LS-Extrakt, β-Caroten, Lithospermum-Extrakt.

Von Interesse ist auch die Zugabe von Duftstoffen. Den erfindungsgemäßen Stiften können Anteile von Parfümen zugesetzt werden, meist gelöst in Alkoholen und als Konzentrat vorliegend.

Weiterhin von besonderem Interesse ist die Zugabe von Farbstoffen und Pigmenten zu den erfindungsgemäßen Stiften. Es können alle bekannten organischen Farbstoffe und anorganischen Pigmente verwendet werden, die in der Kosmetik üblich sind. Dabei ist zu beachten, daß zur Beibehaltung einer eventuellen Transparenz einer Zusammensetzung die Einfärbung durch organische öllösliche Farbstoffe erforderlich ist, während für transluzente oder noch stärker eingetrübte Zusammensetzungen auch anorganische Pigmente verwendet werden können.

Die organischen öllöslichen Farbstoffe können der Zusammensetzung problemlos hinzugefügt werden. Die Verarbeitung mit anorganischen Pigmenten erfolgt vorteilhaft in der Weise, daß das Pigment oder Pigmentgemisch mit einem Öl vermahlen wird und dann der Zusammensetzung hinzugesetzt wird. Geringe Mengen Pigmente, etwa im Bereich von 0,1 bis 0,3 Gew-%, führen zu farbigen, nahezu transparenten festen Zusammensetzungen, wenn entsprechende Grundstoffe, wie z.B. Lanosterin eingesetzt werden. Bei größeren Pigmentmengen, etwa bei 3 bis 4 Gew-%, ist die Zusammensetzung trübe oder undurchsichtig. Daher ist auch die Formulierung von Lippenstiften, Lippenglanz oder Grundierungen möglich, was ebenfalls besondere Ausführungsformen der Erfindung darstellen, bei denen die Pigimentgehalte bis zu 8 Gew-% betragen können.

Pigmente, Pigmentgemische oder Pulver mit pigmentartiger Wirkung, worunter auch solche mit Perlglanz-Effekt zu verstehen sind, können zum Beispiel umfassen Eisenoxide, Titan(di)oxid, Glimmer, Kaolin, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, verkapselte und unverkapselte Getreidestärken sowie Glimmer-Titanoxid-organischer Farbstoff.

Ein weiteres besonderes Merkmal der Erfindung besteht darin, daß die Zusammensetzung wasserphasenfrei ist, d.h. sie enthält kein gesondert hinzugegebenes Wasser, das als eigenständige Phase aufzufassen wäre. Die Zusammensetzung enthält höchstens solche geringen Wassermengen, die physikalisch gebunden durch einzelne Zuschlagstoffe eingetragen werden. Dieser Anteil liegt jedoch deutlich unter 5 Gew-%. Damit unterscheiden sich die erfindungsgemäßen Zusammensetzungen klar von solchen Präparationen, die Cellulosederivate als Verdickungsmittel in wäßrigen Suspensionen oder Emulsionen enthalten.

Es wurde weiterhin gefunden, daß α-Tocopherylacetat bei den Verarbeitungstemperaturen der Lippenstifte, die im allgemeinen durch das schmelzen der Wachse bei 78-80 °C liegt, und wobei auch die anderen Zusatzstoffe eingearbeitet werden, überraschend eine ausgezeichnete Stabilität aufweist. Das α-Tocopherylacetat ist ein übliches Handelsprodukt (CWS/F von Hoffmann-La Roche, Schweiz).

Zusätzlich zu dem im Anlagerungskomplex mit dem Cellulosederivat vorhandenen α-Tocopherol kann in dem Gemisch der weiteren Zusatzstoffe ein Anteil an α-Tocopherol als Radikalfänger vorhanden sein.

Eine weitere vorteilhafte Ausführungsform der Erfindung enthält in den Zusatzstoffen Mineralsalze. Diese Mineralsalze können mit einem Anteil in der Größenordnung von Spurenelementen in Lebensmitteln oder Mineralwässern vorhanden sein, wobei der Anteil auf die Gesamtmasse des Lippenstiftes bezogen ist. Zu diesen Mineralsalzen gehören Na, K, Ca, P, Fe, I, Cu, Co, Mo und Zn sowie Cl und SO₄. Derartige Spurenelemente liegen meist in Bereich von 1 - 2,5 g/l Gesamtgehalt vor und können in dieser Größenordnung insgesamt auch in der erfindungsgemäßen Lippenstiftmasse enthalten sein.

Die Herstellung der festen Zusammensetzungen erfolgt erfindungsgemäß in der Weise, daß zuerst das Cellulosederivat in einer Ölphase dispergiert wird unter Zusatz von Phospholipiden wie Phosphatidylcholin bei ca. 40 - 55°C und unter Rühren mit 200-400 U/Min. Dann erfolgt der Zusatz von α-Tocopherolester in Pulverform, und nach einer Temperaturerhöhung auf ca. 60-65 °C wird homogenisiert bei ca. 10.000-15.000 U/min. Danach werden weitere kosmetische Wirkstoffe oder sonstige Zusatzstoffe unter Rühren und bei Temperaturen, die diesen Stoffen angemessen sind und dem Fachmann auf diesem Gebiet bekannt sind, hinzugegeben. Bei Temperaturen zwischen etwa 60 und 80 °C wird das homogene Gemisch, das zuvor in üblicher Weise durch langsames Rühren entlüftet worden ist, in entsprechende Formen gegossen und abgekühlt.

Gegenstand der Erfindung ist auch ein vitaminahltiger Lippen- oder Pflegestift nach den Merkmalen von Anspruch 1, hergestellt nach dem zuvor genannten Verfahren, wobei die Herstellung ohne Zusatz einer Wasserphase durchgeführt wird.

Falls harzartige Polymere und Copolymere als Zusatzstoffe hinzugegeben werden und deren Schmelzpunkt höher liegt, ist es vorteilhaft, derartige Stoffe durch Schmelzen in die Zusammensetzung aufzunehmen.

Die Erfindung soll nachstehend durch Beispiele näher erläutert werden. Alle Angaben erfolgen in Gewichtsprozent, sofern nichts anderes angegeben ist.

| Beispiel 1 **Lippenstift I** | |
|---|---|
| Caprylic/Capric/Isostearic/ adipic triglyceride | 7,0 |
| Isopropyl palmitate | 11,0 |
| Candelilla wachs | 6,5 |
| Ozokerite wachs | 2,5 |
| Carnauba wachs | 0,5 |
| Bienenwachs | 4,0 |
| Lanolin | 7,0 |
| Rizinusöl | ad 100 |
| Benzophenone-3 | 7,0 |
| Butyl-Methoxybenzoylmethane | 3,0 |
| α-Tocopherol/Cellulose-Komplex | 16 |
| Phosphatidylcholin | 1,4 |
| Glycerol | 3,0 |
| Pigmente | 7,0 |
| Duftstoffe | 1,0 |

Carboxymethylcellulose wird in Rizinusöl dispergiert unter Zusatz von Phosphatidylcholin bei ca. 40°C und unter Rühren mit 360 U/Min. Danach erfolgt der Zusatz von α-Tocopherol in Pulverform (1,2 Gew-%, bezogen auf die Gesamtzusammensetzung). Nach einer Temperaturerhöhung auf ca. 62 °C wird homogenisiert mit ca. 12.000 U/min. Die Wachse werden bei ca 85 °C geschmolzen, auf ca. 75 °C abgekühlt und im Vakuum entlüftet. Nacheinander werden die übrigen Bestandteile bei ca. 55 °C zu der Ölphase mit dem Komplex gegeben und homogenisiert. Nach einer Temperaturerhöhung der Wachse auf Schmelztemperatur wird die Ölphase unter Rühren hinzugegeben und das Gemisch für 5 Minuten homogenisiert. Pigmente und Duftstoffe, die zuvor in Rizinusöl dispergiert werden, werden abschließend zugesetzt, und die Gesamtmasse wird entlüftet und in Formen gegossen und abgekühlt.

| Beispiel 2 **Pflegestift** | |
|---|---|
| Isopropyl palmitate | 15,0 |
| Candelilla wachs | 7 |
| Reiswachs | 3 |
| Carnauba wachs | 3,5 |
| Bienenwachs | 2,0 |
| Lanolin | 10,0 |
| Rizinusöl | ad 100 |
| Jojoba öl | 2,0 |
| α-Tocopherol/Cellulose-Komplex mit Phosphatidylcholin | 8,0 |
| Glycerol | 5,0 |
| Geschmacksstoff | 0,5 |
| Duftstoffe | 0,5 |

Die Verarbeitung erfolgt in gleicher Weise wie im Beispiel 1.

## Patentansprüche

1. Vitaminhaltiger Lippen- oder Pflegestift auf Wachsbasis, **dadurch gekennzeichnet, daß** der Stift einen Anlagerungskomplex enthält, bestehend aus einem Cellulosederivat, umfassend Carboxymethylcellulose, Methylcellulose, Hydroxymethylcellulose, Hydroxypropylcellulose und Hydroxypropylmethylcellulose, das eine Teilchengröße von 0,5 bis 100 µm hat, einem Phospholipid und einem α-Tocopherolester, umfassend α-Tocopherylacetat, -succinat, -propionat, -oleat, -linolat, -sorbat, wobei der Anteil des Komplexes 0,5 bis 40 Gew-% beträgt, bezogen auf die Gesamtmasse, zusammen mit kosmetisch üblichen Fetten, Wachsen und Zusatzstoffen, die einen Anteil von 99,5 bis 60 Gew-% haben.

2. Lippenstift nach Anspruch 1, **dadurch gekennzeichnet, daß** der α-Tocopherolester α-Tocopherylacetat ist.

3. Lippenstift nach Anspruch 1, **dadurch gekennzeichnet, daß** zu den Zusatzstoffen Mineralsalze mit einem Anteil in der Größenordnung von Spurenelementen in Lebensmitteln oder Mineralwässern gehören, wobei der Anteil auf die Gesamtmasse des Lippenstiftes bezogen ist.

4. Lippenstift nach Anspruch 1, **dadurch gekennzeichnet, daß** das Phospholipid Phosphatidylcholin ist.

5. Lippenstift nach Anspruch 1, **dadurch gekennzeichnet, daß** der Anteil des Anlagerungskomplexes 2 bis 30 Gew-% beträgt, bezogen auf die Gesamtmasse.

6. Lippenstift nach Anspruch 1, **dadurch gekennzeichnet, daß** der Anteil des Anlagerungskomplexes 5 bis 25 Gew-% beträgt, bezogen auf die Gesamtmasse.

7. Lippenstift nach Anspruch 1, **dadurch gekennzeichnet, daß** weitere Zusatzstoffe ausgewählt sind unter öllöslichen UVA- und UVB-Filtern wie 4-Aminobenzpesäure-Derivate, Ester der Zimtsäure, Benzophenon-Derivate, 3-Benzylidencampher-Derivate, Salicylsäurederivate, Benzimidazolderivate, Bezoylmethanderivate.

8. Lippenstift nach Anspruch 1, **dadurch gekennzeichnet, daß** weitere Zusatzstoffe ausgewählt sind unter TiO₂, SiO₂, ZnO und Gemischen davon.

9. Lippenstift nach Anspruch 1, **dadurch gekennzeichnet, daß** das Cellulosederivat in Form von Mikrokapseln oder eines Gemisches von Mikrokapseln mit Bruchstücken von Mikrokapseln vorliegt.

10. Vitaminhaltiger Lippen- oder Pflegestift auf Wachsbasis, **dadurch gekennzeichnet, daß** der Stift einen Anlagerungskomplex enthält, bestehend aus einem Cellulosederivat, umfassend carboxymethylcellulose, Methylcellulose, Hydroxymethylcellulose, Hydroxypropylcellulose und Hydroxypropylmethylcellulose, das eine Teilchengröße von 0,5 bis 100 µm hat, einem Phospholipid und einem α-Tocopherolester, umfassend α-Tocopherylacetat, -succinat, -propionat, -oleat, -linolat, -sorbat, wobei der Anteil des Komplexes 0,5 bis 40 Gew-% beträgt, bezogen auf die Gesamtmasse, zusammen mit kosmetisch üblichen Fetten, Wachsen und Zusatzstoffen, die einen Anteil von 99,5 bis 60 Gew-% haben, und hergestellt durch Dispergieren des Cellulosederivates in einer Ölphase unter Zusatz des Phospholipids bei einer Temperatur von 40 bis 55 °C und unter Rühren bei 200 bis 400 U/min, Zugabe des α-Tocopherolesters in Pulverform unter Rühren und Temperaturerhöhung auf 60 bis 65 °C, Homogenisieren des Gemisches bei 10.000-15.000 U/min, Zugabe von Fetten, Wachsen und gegebenenfalls weiteren Zusatzstoffen und Gießen des homogenen Gemisches bei 60 bis 80 °C in Formen, wobei die Herstellung ohne Zusatz einer Wasserphase durchgeführt wird.

## Claims

1. A lip or care stick on a wax base which contains vitamins, said stick comprising an addition.complex of a cellulose derivative, a phospholipid and an α-tocopherol ester, which cellulose derivative is selected from the group consisting of carboxymethyl cellulose, methyl cellulose, hydroxymethyl cellulose, hydroxypropyl cellulose and hydroxypropylmethyl cellulose, said derivative having particle sizes between 0.5 and 100 µm, and which α-tocopherol ester is selected from the group consisting of α-tocopheryl acetate, -succinate,
-propionate, -oleate, -linolate, -sorbate, wherein the proportion of said complex being 0.5 to 40 wt. % relative to the overall weight; together with cosmetically conventional fats, waxes and additives having a proportion of 99.5 to 60 wt. %.

2. A lipstick according to claim 1, wherein the α-tocopherol ester is α-tocopheryl acetate.

3. A lipstick according to claim 1, wherein the additives include mineral salts at a proportion comparable to that of trace elements in foodstuff or mineral waters, wherein the proportion is relative to the overall weight of said lipstick.

4. A lipstick according to claim 1, wherein the phospholipid is phosphatidylcholine.

5. A lipstick according to claim 1, wherein the proportion' of the addition complex is 2 to 30 wt. % relative to the overall weight.

6. A lipstick according to claim 1, wherein the proportion of the addition complex is 5 to 25 wt. % relative to the overall weight.

7. A lipstick according to claim 1, wherein further additives are selected among oil-soluble UVA and UVB filters such as 4-aminobenzoic acid derivatives, cinnamic acid esters, benzophenone derivatives, 3-benzylidene camphor derivatives, salicylic acid derivatives, benzimidazol derivatives, benzoylmethane derivatives.

8. A lipstick according to claim 1, wherein further additives are selected among TiO₂, SiO₂, ZnO and mixtures thereof.

9. A lipstick according to claim 1, wherein the cellulose derivative is provided in the form of micro-capsules or a mixture of micro-capsules and micro-capsule fragments.

10. A lip or care stick on a wax base which contains vitamins, said stick comprising an addition complex with a cellulose derivative selected from the group consisting of carboxymethyl cellulose, methyl cellulose, hydroxymethyl cellulose, hydroxypropyl cellulose and hydroxypropylmethyl .cellulose, said derivative having particle sizes between 0.5 and 100 µm, a phospholipid and an α-tocopherol ester selected from the group consisting of α-tocopheryl acetate, -succinate,
-propionate, -oleate, -linolate, -sorbate, the proportion of said complex being 0.5 to 40 wt. % relative to the overall weight, together with cosmetically conventional fats, waxes and additives having a proportion of 99.5 to 60 wt. %, said stick being produced by dispersing said cellulose derivative in an oil phase while adding the phospholipid under stirring at 200 to 400 rpm at a temperature between 40 to 55 °C, adding the α-tocopherol ester in powdery form under stirring and increasing the temperature to 60 to 65 °C, homogenising of the mixture at 10,000 to 15,000 rpm, adding fats, waxes and, if applicable, further additives, and pouring the homogenised mixture at 60 to 80 °C into moulds, and wherein the production is performed without adding an aqueous phase.

## Revendications

1. Bâton de rouge à lèvres ou bâton de soins pour les lèvres contenant des vitamines sur une base de cire, **caractérisé en ce que** le bâton comprend un complexe d'addition, constitué d'un dérivé cellulosique comprenant la carboxyméthylcellulose, la méthylcellulose, l'hydroxyméthyl-cellulose, l'hydroxypropylcellulose et l'hydroxypropyl-méthylcellulose, avec une taille de particule comprise entre 0,5 et 100 µm, constitué d'un phospholipide et d'un d'α-tocophérol ester, comprenant l'α-tocophéryle acétate, l'α-tocophéryle succinate, l'α-tocophéryle propionate, l'α-tocophéryle oléate, l'α-tocophéryle linolate, l'α-tocophéryle sorbate, la proportion du complexe étant de 0,5 à 40 % en poids par rapport à la masse totale ; les graisses, cires et additifs cosmétiques habituels constituant une proportion de 99,5 à 60 % en poids.

2. Bâton de rouge à lèvres selon la revendication 1, **caractérisé en ce que** l'α-tocophérol ester est l'α-tocophéryle acétate.

3. Bâton de rouge à lèvres selon la revendication 1, **caractérisé en ce que** l'on compte parmi les additifs, des sels minéraux selon une proportion de' l'ordre de celle des oligoéléments dans les produits alimentaires ou les eaux minérales, la proportion étant comprise par rapport à la masse totale du rouge à lèvres.

4. Bâton de rouge à lèvres selon la revendication 1, **caractérisé en ce que** le phospholipide est la phosphatidylcholine.

5. Bâton de rouge à lèvres selon la revendication 1, **caractérisé en ce que** la proportion du complexe d'addition est comprise entre 2 et 30 % en poids, par rapport à la masse totale.

6. Bâton de rouge à lèvres selon la revendication 1, **caractérisé en ce que** la proportion du complexe.d'addition est de 5 à 25 % en poids, par rapport à la masse totale.

7. Bâton de rouge à lèvres selon la revendication 1, **caractérisé en ce que** d'autres additifs sont sélectionnés parmi les filtres UVA et UVB solubles dans l'huile tels que les dérivés d'acide 4-aminobenzoïque, l'ester d'acide cinnamique, . les dérivés de benzophénone, les dérivés de 3-benzylidène camphre, les dérivés d'acide salicylique, les dérivés de benzimidazol, les dérivés de bezoylméthane.

8. Bâton de rouge à lèvres selon la revendication 1, **caractérisé en ce que** d'autres additifs sont sélectionnés parmi les TiO₂, SiO₂, ZnO et des mélanges de ceux-ci.

9. Bâton de rouge à lèvres selon la revendication 1, **caractérisé en ce que** le dérivé cellulosique se présente sous la forme de microcapsules ou d'un mélange de microcapsules avec des fragments de microcapsules.

10. Bâton de rouge à lèvres ou bâton de soins pour les lèvres à teneur en vitamine sur une base de cire, **caractérisé en ce que** le bâton comprend un complexe d'addition constitué d'un dérivé cellulosique comprenant la carboxyméthylcellulose, la méthylcellulose, l'hydroxyméthylcellulose, l'hydroxypropyl - cellulose et l'hydroxypropylméthyl-cellulose, présentant une taille de particule comprise entre 0,5 et 100 µm, constitué d'un phospholipide et d'un α-tocophérol ester, comprenant l'α-tocophéryle acétate, l'α-tocophéryle succinate, l'α-tocophéryle propionate, l'α-tocophéryle oléate, l'α-tocophéryle linolate, l'α-tocophéryle sorbate, la proportion du complexe étant de 0,5 à 40 % en poids par rapport à la masse totale ; les graisses, cires et additifs cosmétiques habituels constituant une proportion de 99,5 à 60 % en poids, et produit par dispersion du dérivé cellulosique dans une phase huileuse en ajoutant le phospholipide à une température de 40 à 55° C et en remuant à raison de 200 à 400 U/min, en ajoutant des α-tocophérol esters sous une forme pulvérulente en remuant et en augmentant la température'de 60 à 65° C, en homogénéisant le mélange à 10 000 à 15 000 U/min, en ajoutant des graisses, cires et éventuellement d'autres additifs et en versant le mélange homogène à une température comprise entre 60 et 80° C dans des moules, la production étant effectuée sans ajout d'une phase aqueuse.
